# EUROPÄISCHE PATENTANMELDUNG

(11) **EP 4 821 442 A1**
(43) Veröffentlichungstag der Anmeldung: **14.10.2026**
(21) Anmeldenummer: 25169686.0
(22) Anmeldetag: 10.04.2025
(51) Int. Cl.: A61B 6/42, A61B 6/00

(54) **DETEKTORHALTERUNGSEINRICHTUNG FÜR EINE RÖNTGENEINRICHTUNG UND RÖNTGENEINRICHTUNG**

(71) Anmelder: Siemens Healthineers AG, 91301 Forchheim (DE)
(72) Erfinder: Anti, Sandra, 90763 Fürth (DE); Dippl, Thomas, 92690 Pressath (DE); Fuhrmann, Michael, 91074 Herzogenaurach (DE); Herold, Sophia Katharina, 96275 Marktzeuln (DE); Hofmann, Jörg, 91301 Forchheim (DE); Limmer, Andreas, 90768 Fürth (DE); Oepping, Susanne, 91088 Bubenreuth (DE); Rupprecht, Florian, 85053 Ingolstadt (DE); ten Cate, Gerben, 91353 Hausen OT Wimmelbach (DE)
(74) Vertreter: Siemens Healthineers Patent Attorneys

(57) **Zusammenfassung**

Die Erfindung betrifft eine Detektorhalterungseinrichtung (1) für eine Röntgeneinrichtung (30, 38), aufweisend einen Röntgendetektor (2) und eine Stativanordnung (9), von welcher der Röntgendetektor (2) innerhalb eines Gehäuses (4) eines Detektormoduls (3) getragen wird, wobei der Röntgendetektor (2) eine sich in einer Detektorebene erstreckende Detektorfläche, die hinter einer röntgendurchlässigen Wand (5) des Gehäuses (4) angeordnet ist, aufweist, dadurch gekennzeichnet, dass der Röntgendetektor (2) mittels wenigstens eines ansteuerbaren Verschiebeaktors (8) in wenigstens einer Richtung (6, 7) in der Detektorebene innerhalb des Gehäuses (4) bewegbar ist.

## Beschreibung

Die Erfindung betrifft eine Detektorhalterungseinrichtung für eine Röntgeneinrichtung, aufweisend einen Röntgendetektor und eine Stativanordnung, von welcher der Röntgendetektor innerhalb eines Gehäuses eines Detektormoduls getragen wird, wobei der Röntgendetektor eine sich in einer Detektorebene erstreckende Detektorfläche, die hinter einer röntgendurchlässigen Wand, insbesondere Platte, des Gehäuses angeordnet ist, aufweist. Daneben betrifft die Erfindung eine Röntgeneinrichtung.

Moderne Röntgeneinrichtungen zur Radiographie sollen verschiedene Arten von Aufnahmetechniken unterstützen, beispielsweise sowohl Aufnahmen bei stehendem Patienten als auch Aufnahmen bei liegendem und sitzendem Patienten. Um solche Flexibilität zu erreichen, werden Radiographie-Röntgeneinrichtungen üblicherweise in mehreren Komponenten bereitgestellt. Derartige Komponenten umfassen zunächst eine Strahlerhalterungseinrichtung zur Halterung eines Röntgenstrahlers, welche beispielsweise zur möglichst effizienten Raumausnutzung an einer Decke eines Raumes, in dem die Röntgeneinrichtung angeordnet ist, montiert werden kann. Als Detektorhalterungseinrichtung zur Erstellung von Röntgenbildern stehender oder sitzender Patienten sind sogenannte Rasterwandgeräte (englisch "bucky wall stand" (BWS)) im Stand der Technik bereits bekannt. Diese umfassen üblicherweise eine Stativanordnung, beispielsweise mit einer Säule, die sich vertikal erstreckt und an der ein Detektormodul höhenverstellbar angeordnet ist. Insbesondere wird das Detektormodul mittels eines Schlittens an einer Führung verfahren, die sich über die Höhe der Säule erstreckt. Das Detektormodul umfasst hierbei üblicherweise eine für Röntgenstrahlung durchlässige Wand, hinter der der Röntgendetektor (Bildempfänger), heutzutage üblicherweise ein digitaler Röntgendetektor, angeordnet ist. Die röntgendurchlässige Wand ist plattenartig ausgebildet und kann daher auch als Platte bezeichnet werden. Zwischen dem Röntgendetektor und der röntgendurchlässigen Wand ist häufig auch ein Streustrahlenraster vorgesehen. Das Detektormodul kann ferner Dosismesskammern, insbesondere für eine Belichtungsautomatik, umfassen.

Die Hubbewegung des Detektormoduls an der Säule kann hierbei manuell und/oder automatisch, beispielsweise mittels eines Motors oder sonstigen geeigneten Aktors, erreicht werden. Bekannt ist es auch, das Detektormodul kippbar, beispielsweise mittels einer Kippeinheit, an der Säule anzuordnen, um das Detektormodul und somit den Röntgendetektor in einem Bereich verkippen zu können, beispielsweise um +90° bis -20°. Bei einer Kippung von +90° ist die röntgendurchlässige Wand/Platte genau wie die Detektorfläche horizontal ausgerichtet, sodass beispielsweise Arm- oder Handaufnahmen bei sitzenden Patienten möglich sind. Fuß- und Beinaufnahmen sind ebenso möglich. Die andere Extremstellung wird für Spezialaufnahmen verwendet. An dem Detektormodul können Handgriffe, an denen sich ein Patient festhalten kann, angeordnet sein. Es wurde auch bereits vorgeschlagen, an einem Rasterwandgerät Röntgenbilder anzufertigen, die einen größeren Bereich als die Detektorfläche des Röntgendetektors abdecken und aus Einzelbildern zusammengesetzt werden, indem das Detektormodul mit dem Röntgendetektor entlang des Patienten in Höhenrichtung für die verschiedenen Einzelbilder positioniert wird. Bei derartigen Aufnahmevorgängen oder sonstigen Anwendungen, in denen eine Bewegung des Detektormoduls stattfindet, wird üblicherweise eine Patientenhilfseinrichtung bereitgestellt, die den Patienten beim Einnehmen der korrekten Aufnahmeposition und bei deren Halten unterstützt. Eine solche Patientenhilfseinrichtung kann beispielsweise Stufen und/oder einen Stuhl, eine zusätzliche, vor dem bewegten Detektormodul platzierte Wand und besondere Haltegriffe umfassen. Allerdings bildet eine solche Patientenhilfseinrichtung ein zusätzliches Zubehör, welches die Röntgeneinrichtung zudem komplexer gestaltet.

Insbesondere bei gekippten Stellungen des Detektormoduls, aber auch dann, wenn zusätzliche Hilfsmittel wie die genannte Patientenhilfseinrichtung verwendet werden, kann es leicht zu Kollisionen zwischen der Stativanordnung, insbesondere der Säule, und anderen Komponenten, insbesondere Teilen der Strahlerhalterungseinrichtung, kommen. Dies schränkt den Arbeitsbereich ein und erfordert hohe Aufmerksamkeit des Personals, um Kollisionen zu vermeiden. Bei Aufnahmen mit vertikal stehendem Detektormodul muss der Patient in Querrichtung genau positioniert werden, da häufig der Untersuchungsbereich die gesamte Detektorfläche nutzt.

Doch auch weitere Probleme hinsichtlich der heute bekannten Rasterwandgeräte bestehen. So ist die Höhe des Rasterwandgeräts genau wie die Hubbewegung des Rasterwandgeräts, insbesondere aufgrund der Ausgestaltung und Größe des Detektormoduls, limitiert. Nachdem die Stativanordnung auf dem Boden des Raumes, in dem die Röntgeneinrichtung installiert ist, fest montiert ist, muss bei quer am Patienten versetzten Untersuchungsbereichen eine Neupositionierung des Patienten erfolgen. Die Detektorgröße ist hierbei und allgemein im Detektoreinschub limitiert.

Auch in der Handhabung bekannter Rasterwandgeräte bestehen Schwierigkeiten. So ist die Reinigung einer solchen Detektorhalterungseinrichtung durch zusätzliches Zubehör und mit bekannten Verkleidungselementen schwierig. Das Streustrahlenraster muss manuell eingelegt und, falls es nicht benötigt wird, entfernt werden. Bei einer manuell möglichen Bewegung des Detektormoduls an der Stativanordnung, insbesondere der Säule, ist ein Gewichtsausgleich erforderlich. Eine hohe Bauraumanforderung ergibt sich insbesondere, wenn ein sogenannter Distanzkasten in horizontaler Richtung zwischen der Stativanordnung, insbesondere Säule, und dem Detektormodul vorgesehen ist, um beispielsweise eine Patientenlagerungseinrichtung geeignet zu dem Detektormodul platzieren zu können. Bei manueller Betätigung der vorgesehenen Freiheitsgrade ist kein automatischer Workflow möglich.

Bei bekannten Röntgeneinrichtungen sind zudem häufig zusätzlich zu der Detektorhalterungseinrichtung, also dem Rasterwandgerät, Patiententische als Patientenlagerungseinrichtung vorgesehen, die ebenso zumindest eine Aufnahme bzw. einen Einschub für einen Röntgendetektor und ein manuelles Streustrahlenraster enthalten können, um Aufnahmen bei liegendem Patienten durchführen zu können.

Vor diesem Hintergrund liegt der Erfindung die Aufgabe zugrunde, eine demgegenüber verbesserte Detektorhalterungseinrichtung und Röntgeneinrichtung zu schaffen, welche zumindest einen Teil der genannten Probleme löst und bei einfachem Aufbau und einfacher Handhabung flexible Einsatzmöglichkeiten, wie beispielsweise automatische Workflows, bietet.

Zur Lösung dieser Aufgabe sind erfindungsgemäß eine Detektorhalterungseinrichtung und eine Röntgeneinrichtung mit den Merkmalen der nebengeordneten Ansprüche vorgesehen. Vorteilhafte Weiterbildungen ergeben sich aus den Unteransprüchen.

Bei einer Detektorhalterungseinrichtung der eingangs genannten Art ist erfindungsgemäß vorgesehen, dass der Röntgendetektor mittels wenigstens eines ansteuerbaren Verschiebeaktors in wenigstens einer Richtung in der Detektorebene innerhalb des Gehäuses bewegbar ist.

Es wird ein Gehäuse vorgeschlagen, dass die röntgendurchlässige Wand, insbesondere Platte, bereitstellt und dessen Innenraum größer als der darin aufgenommene Röntgendetektor ist, so dass der Röntgendetektor mittels wenigstens eines Verschiebeaktors automatisch entlang wenigstens einer Richtung innerhalb des Gehäuses verschiebbar ist. Insbesondere weist das Gehäuse in der Detektorebene eine erste Breite und eine erste Höhe auf, wobei der Röntgendetektor eine geringere zweite Breite und/oder eine geringere zweite Höhe aufweist. In einer besonders vorteilhaften Ausgestaltung ist dabei vorgesehen, dass der Röntgendetektor mittels des wenigstens einen Verschiebeaktors in einer Höhenrichtung und in einer Breitenrichtung, die senkrecht zueinander stehen, verschiebbar ist.

Die Verschiebbarkeit innerhalb des Gehäuses wird dabei insbesondere durch eine Verschiebeeinrichtung mit dem wenigstens einen Verschiebeaktor bereitgestellt werden. Hierbei können grundsätzlich bekannte Linearstellmittel, beispielsweise Spindeltriebe, und/oder bevorzugt ein geführter Schlitten, an dem der Röntgendetektor angeordnet ist, verwendet werden. Bei der Verstellung in zwei zueinander senkrechten Richtungen kann beispielsweise in der einen Richtung der Schlitten entlang der einen Richtung entlang eines Führungsmittels, beispielsweise wenigstens eines Führungszylinders oder -rohres, verschoben werden, während dieses Führungsmittel selbst, insbesondere beidseitig geführt, senkrecht zu dessen Verlauf linearverstellbar ist. Denkbar ist es auch, einen anhand von Führungsmitteln in einer Richtung verstellbaren Schlitten zu verwenden, wobei der Röntgendetektor auf dem Schlitten in einer dazu senkrechten Richtung verschiebbar ist. Ferner ist es möglich, durch Rotation einer beispielsweise durch ein Führungsmittel hergestellten Bewegungsachse in der Detektorebene ausgehend von einer Ausrichtung in der ersten Richtung wenigstens eine zweite Richtung zur Translation bereitzustellen. Selbstverständlich sind auch andere konkrete Ausgestaltungen denkbar.

Das Gehäuse ist hierbei, insbesondere in der Detektorebene, bevorzugt rechteckig, ggf. auch quadratisch, ausgebildet. In einer Grundstellung, in der die Detektorfläche und die röntgendurchlässige Wand vertikal ausgerichtet sind, kann die Höhenrichtung parallel zu einer Seite des Rechtecks, insbesondere einer längeren Seite, verlaufen, die Breitenrichtung senkrecht dazu parallel zu der anderen, insbesondere kürzeren Seite. Anders gesagt erstrecken sich die Höhenrichtung und die Breitenrichtung zumindest in einer Grundstellung entlang Kanten des vorzugsweise rechteckigen Gehäuses.

Es wird also vorgeschlagen, den Röntgendetektor praktisch unsichtbar innerhalb eines Gehäuses automatisch mittels wenigstens eines Verschiebeaktors zu verschieben, so dass bei Nutzung dieses wenigstens einen, bevorzugt dieser wenigstens zwei, Freiheitsgrade nach außen keine bewegten Teile vorliegen und insbesondere das Detektormodul selbst nicht bewegt wird. Dies bringt eine Vielzahl von Vorteilen mit sich.

So ist bei einem (insbesondere die Ansteuerbarkeit des Verschiebeaktors nutzenden und somit automatisch durchführbaren) Panoramabetrieb, bei dem mehrere Einzelbilder zu einem Röntgenbild zusammengesetzt werden, keine Bewegung des Detektormoduls oder des Patienten selbst mehr notwendig. Beispielsweise kann vorgesehen sein, dass die Steuereinrichtung der Röntgeneinrichtung in einem Panoramabetrieb zum Ansteuern des Verschiebeaktors zur Bewegung des Röntgendetektors in verschiedene Positionen entlang wenigstens einer der wenigstens einen Richtung und zur Ansteuerung des Röntgenstrahlers und des Röntgendetektors zur Aufnahme von Einzelbildern eines Panoramabildes an den Positionen ausgebildet ist. Bei einer Verschiebbarkeit in Höhenrichtung und in Breitenrichtung sind derartige Panoramaaufnahmen in beiden Richtungen denkbar. Insbesondere ist es auch bei anderen Aufnahmefolgen neben dem Panoramabetrieb in Höhenrichtung und/oder Breitenrichtung, entsprechend der Querrichtung in der Grundstellung mit vertikal ausgerichteter Detektorfläche, nicht länger erforderlich, den Patienten aufwendig umzuplatzieren. Die vorliegende Erfindung erlaubt mithin einen größeren bzw. erweiterten Bildgebungsbereich. Zudem sind auch, insbesondere bei Verschiebung entlang von zwei Richtungen/Achsen innerhalb des Gehäuses, neue Aufnahmemodi denkbar, beispielsweise solche, die Panoramen hinsichtlich von zwei Richtungen erlauben.

Die Verschiebung des Röntgendetektors innerhalb des Gehäuses, also insbesondere hinter der röntgendurchlässigen Wand bzw. Platte des Gehäuses, ist in mehrerlei weiterer Hinsicht vorteilhaft. So sind Risiken durch bewegte Teile reduziert. Zusätzliche Patientenhilfseinrichtungen ("Ortho-Supports"), die aufgrund eines bewegten Detektormoduls Stufen, Haltegriffe und sonstige Unterstützung bereitstellen, sind nicht länger erforderlich. Es wird eine Verbesserung der Zugänglichkeit für Menschen mit eingeschränkter Mobilität erreicht. Nachdem mehr geschlossene, plattenartige Teile, wie beispielsweise das Gehäuse, vorliegen, kann die Detektorhalterungseinrichtung auch verbessert gereinigt werden.

Es sei noch angemerkt, dass auch nicht an einer Wand des Raumes, in dem die Röntgeneinrichtung installiert ist, befestigte Röntgendetektorhalterungen häufig als "Rasterwandgerät" oder englisch "bucky wall stand" bzw. "bucky stand" bezeichnet werden. In diesem Sinne lässt sich auch die Detektorhalterungseinrichtung der vorliegenden Erfindung als ein Rasterwandgerät verstehen, welches dessen Funktionen vollständig bereitstellt. In jedem Fall jedoch ist die beschriebene Detektorhalterungseinrichtung ein "bucky stand", auch wenn, wie im Folgenden noch beschrieben, durch erhöhte Flexibilität der Einsatzbereich erweitert werden kann.

Innerhalb des Gehäuses kann, wie grundsätzlich bekannt, auch wenigstens eine Dosismesskammer verbaut sein. Die Position der wenigstens ein Dosismesskammer kann außen auf der röntgendurchlässigen Wand, wie grundsätzlich bekannt, markiert werden.

Zweckmäßigerweise kann in dem Gehäuse ein dem Röntgendetektor zugeordnetes Streustrahlenraster vorgesehen sein, welches zumindest mit dem Röntgendetektor bewegbar ist. Wie bereits bekannt, kann das Detektormodul also auch weiterhin wenigstens ein Streustrahlenraster umfassen, welches zumindest mit dem Röntgendetektor bewegt werden kann. Dabei ist die Bewegung mit dem Röntgendetektor vorliegend so zu verstehen, dass das Streustrahlenraster entweder mit dem Röntgendetektor bewegungsgekoppelt oder bewegungskoppelbar ist, also über den Verschiebeaktor bewegbar ist, und/oder mittels eigener, entsprechend angesteuerter Aktorik, insbesondere also wenigstens einem Rasteraktor, bewegbar ist.

Eine konkrete, zweckmäßige Weiterbildung in diesem Zusammenhang kann vorsehen, dass das Streustrahlenraster mittels wenigstens eines Rasteraktors unabhängig von dem Röntgendetektor in der wenigstens einen Richtung bewegbar ist. Dies ermöglicht es, die Position des Streustrahlenrasters innerhalb des Gehäuses auch unabhängig von der Position des Röntgendetektors zu wählen, insbesondere das Streustrahlenraster aus dem Strahlengang der Röntgenstrahlung zu der Detektorfläche zu entfernen, wenn es nicht benötigt wird, oder bei mehreren Streustrahlenrastern wahlweise eines davon in den Strahlengang einzubringen. Konkret kann beispielsweise vorgesehen sein, dass die Steuereinrichtung der Röntgeneinrichtung ausgebildet ist, das Streustrahlenraster durch Ansteuerung des Rasteraktors bei Bildgebungsvorgängen, die ohne Streustrahlenraster durchzuführen sind, in eine die Detektorfläche nicht überdeckende Nichtbenutzungsposition zu verfahren. Auf diese Weise ist keine manuelle Handhabung des Streustrahlenrasters mehr notwendig, sodass eine deutliche Verbesserung des Komforts diesbezüglich gegeben ist.

Hinsichtlich eines Einsetzens, Wechselns oder Entnehmens des Streustrahlenrasters kann in einer zweckmäßigen Ausgestaltung vorgesehen sein, dass das Detektormodul eine Präsentatoranordnung zum Einsetzen und Entnehmen des Streustrahlenrasters aufweist. Die Präsentatoranordnung kann beispielsweise eine aus dem Gehäuse ausfahrbare Schublade oder sonstige Ausfahrvorrichtung (also ausfahrbare und/oder ausziehbare Vorrichtung) umfassen, aus der das Streustrahlenraster entnehmbar bzw. in die es einsetzbar ist. Insbesondere kann das Streustrahlenraster mittels des wenigstens einen Rasteraktors in eine Präsentatorposition innerhalb der Schublade bzw. Ausfahrvorrichtung gefahren werden, um dort präsentiert zu werden. Die Präsentatoranordnung kann ein Greifmittel umfassen, um das Streustrahlenraster für das Ausfahren zu greifen. Die Präsentatoranordnung kann, genau wie der Rasteraktor, von einer Steuereinrichtung ansteuerbar sein, beispielsweise in Reaktion auf die Betätigung eines Bedienelements der Röntgeneinrichtung, die die Präsentation des Streustrahlenrasters anfordert. Beispielsweise kann die Präsentation vergleichbar mit der Präsentation eines Datenträgers bei einem Computer, beispielsweise einer CD, vorgenommen werden.

Wie bereits erwähnt, ist die Detektorhalterungseinrichtung insbesondere derart ausgebildet, dass zumindest in einer Grundstellung die Detektorfläche vertikal ausgerichtet ist. Diesbezüglich wirkt sie mithin wie ein Rasterwandgerät bzw. "bucky stand" und kann beispielsweise für Thorax-Aufnahmen, für die ein Rasterwandgerät am häufigsten genutzt wird, und dergleichen eingesetzt werden. Bevorzugte Weiterbildungen der vorliegenden Erfindung sehen jedoch vor, dass die Detektorhalterungseinrichtung weitere, insbesondere automatisch ansteuerbare, Freiheitsgrade bereitstellt, die die Flexibilität des Einsatzes der Detektorhalterungseinrichtung deutlich erhöhen.

Eine besonders bevorzugte Weiterbildung der vorliegenden Erfindung sieht vor, dass die Stativanordnung eine teleskopartig mittels eines ansteuerbaren Hubaktors höheneinstellbare Säule umfasst, die das Detektormodul trägt. Es wird mithin vorgeschlagen, eine Teleskopsäule zu verwenden, bei der eine äußerst gute Platzausnutzung gegeben ist und insbesondere der Raum oberhalb des Detektormoduls grundsätzlich frei bleibt, sodass beispielsweise andere Komponenten der Röntgeneinrichtung und/oder Hilfsmittel verbessert angeordnet werden können. Insbesondere, worauf im Folgenden noch näher eingegangen werden wird, erlaubt die Nutzung einer teleskopierbaren Säule auch die Anordnung des Detektormoduls unterhalb von Patientenlagerungseinrichtungen. Im Gegensatz zu einer herkömmlichen Säule, in der ein Schlitten mit dem Detektormodul geführt ist, "verschwindet" der mögliche Verstellweg innerhalb der Säule, indem sich diese bei geringerer gewünschter Sollhöhe teleskopartig zusammenzieht und somit das Detektormodul nicht überragen muss.

Dabei kann die Teleskopsäule bei einer Verschiebbarkeit des Röntgendetektors im Gehäuse in Höhenrichtung auch kleiner dimensioniert werden, also mit einer geringeren Maximalhöhe, da ein Teil einer Sollhöhe des Röntgendetektors auch durch die Verschiebung innerhalb des Gehäuses bereitgestellt werden kann. Beispielsweise kann in diesem Kontext eine Steuereinrichtung der Röntgeneinrichtung ausgebildet sein, zur Einstellung einer Sollhöhe des Röntgendetektors einen Teil der Sollhöhe über den Verschiebeaktor und einen Teil der Sollhöhe über den Hubaktor bereitzustellen. Insbesondere ist für wenigstens einen Betriebsmodus, insbesondere bei der Einzelbildaufnahme, eine maximale Ausnutzung des durch den Verschiebeaktor bereitstellbaren Weges denkbar.

In einer zweckmäßigen Weiterbildung der vorliegenden Erfindung kann ferner vorgesehen sein, dass das Detektormodul über eine Linearverstelleinrichtung mit wenigstens einem ansteuerbaren Linearaktor in wenigstens einer horizontalen Richtung verstellbar mit der Säule gekoppelt ist. Beispielsweise kann also eine zusätzliche Längsverschiebung als weiterer, die Flexibilität erhöhender Freiheitsgrad bereitgestellt werden.

Vorzugsweise kann die Detektorhalterungseinrichtung mittels einer Verankerungsanordnung in einem Boden eines Raumes, in dem die Röntgeneinrichtung installiert ist, verankerbar sein. Beispielsweise kann die Stativanordnung also einen, insbesondere die Teleskopsäule tragenden, Sockel aufweisen, der beispielsweise mittels hinreichend kräftiger Verschraubungen in dem Boden verankert ist.

Grundsätzlich denkbar ist jedoch auch eine Bewegbarkeit der Detektorhalterungseinrichtung an sich. In solchen Ausführungsbeispielen kann vorgesehen sein, dass die Detektorhalterungseinrichtung eine Verankerungsanordnung, insbesondere umfassend ein Schienensystem, zur in zumindest einer horizontalen Richtung verschiebbaren Verankerung der Stativanordnung in einem Boden aufweist.

In einer besonders bevorzugten Weiterbildung der Erfindung ist vorgesehen, dass das Detektormodul mittels einer Kippeinrichtung zumindest zwischen einer oder der Grundstellung, in der die Detektorfläche vertikal ausgerichtet ist, und einer Horizontalstellung, in der die Detektorfläche horizontal ausgerichtet ist, kippbar mit der Stativanordnung gekoppelt ist, wobei die Kippeinrichtung insbesondere einen ansteuerbaren, die Kippung bewirkenden Kippaktor aufweist. Das Detektormodul und mithin die Detektorfläche hinter der röntgendurchlässigen Wand können also zumindest auch in eine Horizontalstellung gebracht werden, indem mittels entsprechender Kippmittel um eine zur Detektorfläche parallele, horizontale Kippachse gekippt wird. Statt der vertikalen Grundstellung ergibt sich dann eine horizontale Horizontalstellung, in der beispielsweise ein aufzunehmendes Körperteil auf die röntgendurchlässige Wand aufgelegt werden kann, beispielsweise bei Hand- und/oder Armaufnahmen sitzender Patienten. Mit besonderem Vorteil jedoch kann, insbesondere bei Verwendung einer Teleskopsäule der Stativanordnung, vorgesehen sein, dass eine Patientenlagerungseinrichtung, beispielsweise ein einfacher Trolley oder ein einfacher Stretcher, gegebenenfalls auch ein Patiententisch, über die röntgendurchlässige Wand, insbesondere das gesamte Detektormodul, gefahren werden, sodass die Detektorhalterungseinrichtung beispielsweise auch für Aufnahmen an einem liegenden Patienten eingesetzt werden kann und ein weiterer Röntgendetektor in einem Patiententisch gegebenenfalls nicht mehr benötigt wird. Mit anderen Worten kann die Detektorhalterungseinrichtung auf diese Weise eine Art Zwei-in-Eins-Lösung bieten, da sie nicht nur als Rasterwandgerät fungiert, sondern zusätzlich auch bei liegenden Patienten in Kombination mit einer Patientenlagerungseinrichtung eingesetzt werden kann. Dies ermöglicht es beispielsweise, statt einem aufwendigen Patiententisch, der beispielsweise einen Einschub für ein Röntgendetektor oder ein Röntgendetektor selbst aufweisen könnte, eine deutlich einfachere Patientenlagerungseinrichtung, beispielsweise einen einfachen Trolley, einzusetzen. Bevorzugt im Hinblick auf die mögliche Automatisierung von Workflows kann die Kippeinrichtung einen durch die Steuereinrichtung der Röntgeneinrichtung ansteuerbaren Kippaktor aufweisen.

Es sei angemerkt, dass in Ausführungsbeispielen durch die Kippeinrichtung bzw. konkret den Kippaktor neben der Grundstellung und der Horizontalstellung auch weitere Kippstellungen einstellbar sein können, beispielsweise eine Kippstellung um -20° für Spezialaufnahmen und dergleichen.

Um einen weiteren, die Flexibilität des Einsatzes weiter erhöhenden Freiheitsgrad bereitzustellen, kann vorgesehen sein, dass das Detektormodul über eine Schwenkeinrichtung, die ein Verschwenken des Detektormoduls um eine zur Detektorfläche senkrechte Schwenkachse erlaubt, mit der Stativanordnung gekoppelt ist, wobei die Schwenkeinrichtung insbesondere einen ansteuerbaren, die Verschwenkung bewirkenden Schwenkaktor aufweist. Dies ist insbesondere dann zweckmäßig, wenn das Gehäuse parallel zur Detektorebene eine Rechteckform aufweist, in der eine Ausdehnung, insbesondere die erste Höhe, größer, insbesondere deutlich größer, als eine zweite Ausdehnung, insbesondere die erste Breite, ist. Das bedeutet nämlich, dass in der Grundstellung eine deutlich weitergehende Verschiebbarkeit des Röntgendetektors in der Höhenrichtung möglich ist als in der Breitenrichtung (so überhaupt vorgesehen). Sollen jedoch Einzelbilder, beispielsweise bei einer Panoramaaufnahme, in der Breitenrichtung aufeinanderfolgen und wäre die Breitenrichtung in dieser Richtung nicht ausreichend, um alle Aufnahmepositionen abzudecken, kann eine Verschwenkung um die zur Detektorfläche senkrechte Schwenkachse, insbesondere um 90°, dazu führen, dass dies doch ermöglicht wird. Eine Umpositionierung des Patienten ist dann nicht nötig. Insbesondere existiert also neben der Grundstellung wenigstens eine weitere Schwenkstellung, in der der Röntgendetektor um 90° verschwenkt ist, wobei auch dazwischen liegende oder darüber hinaus gehende Schwenkstellungen denkbar sein können. Insbesondere kann in der weiteren Schwenkstellung die längere Kante des Gehäuses horizontal verlaufen, während sie in der Grundstellung vertikal verläuft. Bevorzugt ist auch hier ein von der Steuereinrichtung ansteuerbarer Schwenkaktor vorgesehen, um Workflows zu automatisieren und zu vereinfachen.

Eine Schwenkeinrichtung ist insbesondere auch dann vorteilhaft, wenn eine Verwendung der Detektorhalterungseinrichtung für Lateralaufnahmen bei liegenden Patienten vorgesehen ist, bei denen der zumindest im Wesentlichen vertikal stehende Röntgendetektor seitlich neben dem Patienten bzw. der entsprechenden Patientenlagerungseinrichtung angeordnet wird. Im Gegensatz zum stehenden Patienten verläuft dessen Längsachse dann horizontal, sodass zur Aufnahme einer Bilderfolge entlang der Längsachse des Patienten zweckmäßigerweise auch die lange Kante des Gehäuses horizontal ausgerichtet ist und mithin der längst mögliche Verstellweg in der wenigstens einen Richtung, hier insbesondere der Höhenrichtung des Detektormoduls, bereitgestellt wird. Dies ist mittels der Schwenkeinrichtung möglich.

Dabei ist es bei Vorsehen sowohl der Schwenkeinrichtung als auch der Kippeinrichtung und der Linearverstelleinrichtung oder von zwei dieser Einrichtungen letztlich irrelevant, in welcher Reihenfolge diese zwischen dem Detektormodul und der Stativanordnung, insbesondere der Säule, verbaut sind. Bevorzugt ist von dem Detektormodul ausgehend zunächst die Schwenkbarkeit, dann die Kippbarkeit, dann, falls vorgesehen, die Linearverstellbarkeit realisiert. Es versteht sich, dass, insbesondere bei auch manueller jeweiliger Verstellmöglichkeit, der Schwenkeinrichtung, der Kippeinrichtung und der Linearverstelleinrichtung jeweilige Arretiermittel zur Arretierung des Detektormoduls in der jeweiligen Stellung zugeordnet sein können.

In zweckmäßiger Weiterbildung kann vorgesehen sein, dass das Detektormodul, insbesondere das Gehäuse, wenigstens einen Haltegriff für eine Untersuchungsperson, mithin einen Patienten, aufweist. Derartige Haltegriffe, die auch von dem Stand der Technik bekannten Rasterwandgeräten bereits bekannt sind, können auch im Rahmen der vorliegenden Erfindung nutzbringend vorgesehen werden, um das Einnehmen und/oder Halten einer Stellung bei der Aufnahme von Röntgenbildern zu unterstützen. Insbesondere lassen sich diese Haltegriffe bzw. das Gehäuse an sich, aufgrund der Verschiebung des Röntgendetektors innerhalb des Gehäuses, auch dann uneingeschränkt weiter verwenden, wenn Aufnahmevorgänge mit bewegtem Röntgendetektor vorliegen.

In diesem Zusammenhang sei noch darauf hingewiesen, dass die erfindungsgemäße Detektorhalterungseinrichtung als Rasterwandgerät bzw. Substitut für ein Rasterwandgerät bevorzugt nicht ausgestaltet ist, einen Erwachsenen vollständig zu tragen, wie dies beispielsweise bei einem Patiententisch der Fall wäre. Beispielsweise kann vorgesehen sein, dass eine Maximalbelastung der Detektorhalterungseinrichtung weniger als 60 kg, insbesondere weniger als 50 kg, wie dies für stehende Patienten mit Handgriffen üblich ist, betragen kann.

In beispielhaften konkreten Ausgestaltungen kann vorgesehen sein, dass das Gehäuse in der Detektorebene eine Ausdehnung von 50 bis 100 mal 50 bis 150 cm aufweist und/oder der Röntgendetektor in der Detektorebene eine Ausdehnung von 20 bis 50 mal 20 bis 80 cm aufweist. Beispielsweise ist ein Gehäuse der Größe 60 mal 120 cm, insbesondere Breite mal Höhe, denkbar, in dem ein Detektor mit einer Detektorfläche einer Größe von 25 mal 50 cm bewegbar ist. In einem anderen Beispiel kann das Gehäuse 81 mal 144 cm (insbesondere Breite mal Höhe) groß sein, während der Röntgendetektor bzw. die Detektorfläche 30 mal 53 cm groß sein kann.

Besonders vorteilhaft ist es, wenn alle Aktoren, die zur Realisierung der Freiheitsgrade verwendet werden, ansteuerbar sind, um, wie teilweise bereits angedeutet, mittels der Steuereinrichtung der Röntgeneinrichtung möglichst weitgehend automatisierte Workflows realisieren zu können.

Eine erfindungsgemäße Röntgeneinrichtung weist eine Strahlerhalterungseinrichtung mit wenigstens einem Röntgenstrahler und wenigstens einem ansteuerbaren Strahleraktor zur Positionierung des Röntgenstrahlers, eine Detektorhalterungseinrichtung gemäß der vorliegenden Erfindung und eine Steuereinrichtung auf. Sämtliche Ausführungen hinsichtlich der Detektorhalterungseinrichtung lassen sich analog auf die Röntgeneinrichtung übertragen und umgekehrt.

Dabei kann die Steuereinrichtung bevorzugt die Aktorik der Detektorhalterungseinrichtung derart ansteuern, dass die Position des Röntgendetektors, insbesondere der Detektorfläche, der Position des Röntgenstrahlers (umfassen selbstverständlich auch dessen Ausrichtung) folgt. Auf diese Weise steht der Röntgendetektor immer richtig zum Röntgenstrahler, sodass das Risiko für fehlerhafte Aufnahmevorgänge deutlich vermindert wird. Insbesondere kann vorgesehen sein, dass die Steuereinrichtung der Röntgeneinrichtung zur aufeinander zum Empfang von Röntgenstrahlung des Röntgenstrahlers mittels des Röntgendetektors abgestimmten Bewegung des Röntgenstrahlers und des Röntgendetektors eingerichtet ist. So ist es insbesondere auch möglich, beispielsweise für Panoramabilder oder dergleichen, sozusagen "Aufnahmetrajektorien" von Aufnahmeposition zu Aufnahmeposition in Gleichklang zu realisieren und eine äußerst weitgehende Automatisierung zu erreichen.

In einer besonders zweckmäßigen Weiterbildung der vorliegenden Erfindung weist die Röntgeneinrichtung ferner wenigstens einen Patiententisch und/oder wenigstens eine mobile Patientenlagerungseinrichtung auf. Insbesondere, wie oben bereits dargelegt, ist dabei die Detektorhalterungseinrichtung so ausgebildet, beispielsweise durch das Vorsehen der Kippbarkeit, dass der Röntgendetektor der Detektorhalterungseinrichtung auch für Aufnahmen bei auf der mobilen Patientenlagerungseinrichtung bzw. dem Patiententisch liegendem Patienten genutzt werden kann. Mit besonderem Vorteil ist hierbei vorgesehen, dass die Detektorhalterungseinrichtung derart dimensioniert ist, dass in wenigstens einer Einstellung mit dem Detektormodul in der Horizontalstellung eine Liegefläche des Patiententischs und/oder der mobilen Patientenlagerungseinrichtung über der röntgendurchlässigen Wand des Gehäuses platzierbar ist. Besonders zweckmäßig weist die Stativanordnung dann die beschriebene Teleskopsäule auf, sodass sich die Detektorhalterungseinrichtung sozusagen kleinstmöglich zusammenzieht und keine oder zumindest keine relevanten Anteile der Stativanordnung über das Detektormodul hinausragen, sodass der gesamte Bereich oberhalb des Detektormoduls für die Platzierung der Liegefläche des Patiententischs und/oder der mobilen Patientenlagerungseinrichtung, insbesondere eines Trolleys oder Stretchers, zur Verfügung steht. Die Detektorhalterungseinrichtung bildet dann sozusagen eine Zwei-in-Eins-Lösung, die den Röntgendetektor sowohl für Aufnahmen mit liegendem Patienten auf der Patientenlagerungseinrichtung als auch für Aufnahmen bei stehendem Patienten in der Funktion als "bucky stand" bereitstellt.

In einer zweckmäßigen Weiterbildung der vorliegenden Erfindung kann ferner vorgesehen sein, dass die Röntgeneinrichtung wenigstens eine Kamera aufweist, wobei die Steuereinrichtung zur Ansteuerung wenigstens eines Aktors der Röntgendetektorhalterungseinrichtung in Abhängigkeit von Bilddaten der Kamera ausgebildet ist. Aufgrund der vielfältigen, ansteuerbaren Freiheitsgrade der Detektorhalterungseinrichtung lassen sich mithin auch kamerabasierte automatisierte Workflows realisieren, welche beispielsweise bei einer Positionsveränderung des Patienten durch Ansteuerung der Aktorik, insbesondere sowohl der Strahlerhalterungseinrichtung als auch der Detektorhalterungseinrichtung, die Aufnahmeanordnung derart nachfahren kann, dass die Bildaufnahme fortgesetzt werden kann.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: schematisch eine Frontansicht einer erfindungsgemäßen Detektorhalterungseinrichtung mit dem Detektormodul in der Grundstellung,
- Fig. 2: schematisch eine Seitenansicht einer erfindungsgemäßen Detektorhalterungseinrichtung mit dem Detektormodul in der Grundstellung,
- Fig. 3: eine prinzipielle Querschnittsansicht durch das Detektormodul,
- Fig. 4: Teilbilder, die das Detektormodul der Detektorhalterungseinrichtung in verschiedenen Schwenkstellungen zeigen,
- Fig. 5: eine Ansicht eines ersten Ausführungsbeispiels einer erfindungsgemäßen Röntgeneinrichtung in einem ersten Betriebszustand,
- Fig. 6: eine Ansicht des ersten Ausführungsbeispiels in einem zweiten Betriebszustand,
- Fig. 7: eine Ansicht eines zweiten Ausführungsbeispiels einer erfindungsgemäßen Röntgeneinrichtung in einem ersten Betriebszustand, und
- Fig. 8: eine Ansicht des zweiten Ausführungsbeispiels in einem zweiten Betriebszustand.

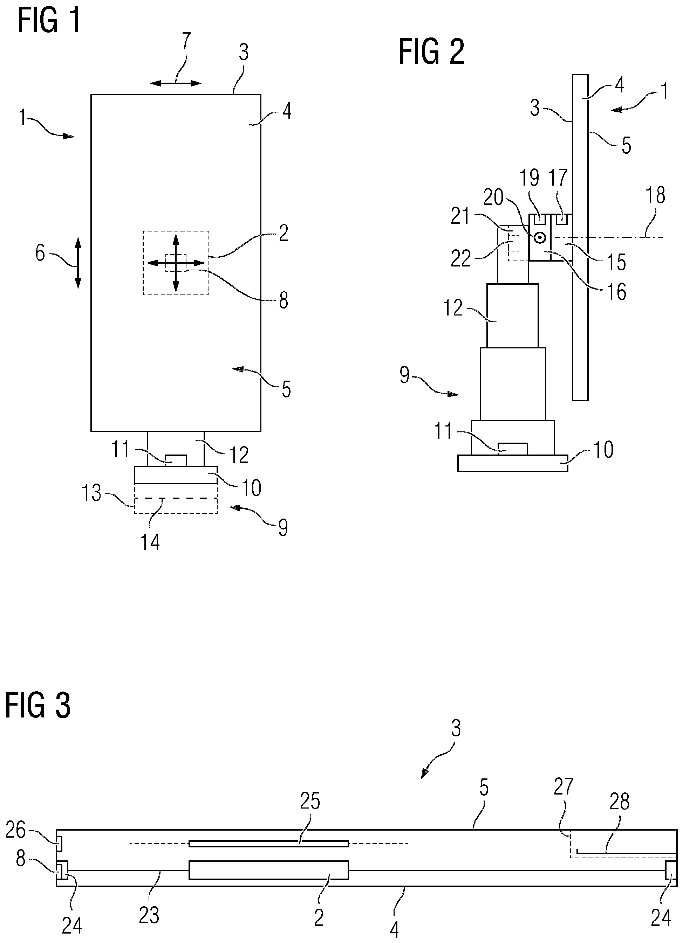

Fig. 1 und Fig. 2 zeigen Prinzipskizzen einer erfindungsgemäßen Detektorhalterungseinrichtung 1 in einer Frontansicht und einer Seitenansicht, wobei sich ein den in Fig. 1 gestrichelt angedeuteten Röntgendetektor 2 beinhaltendes Detektormodul 3, dessen Gehäuse 4 den Röntgendetektor 2 umschließt, in einer Grundstellung befindet. In dieser Grundstellung ist die röntgendurchlässige vordere, plattenartige Wand 5 (Platte), die sich über die Detektorfläche des Röntgendetektors 2 erstreckt, vertikal ausgerichtet. Das bedeutet, eine Detektorebene, in der die Detektorfläche liegt, erstreckt sich ebenso vertikal. Das Gehäuse 4 und mithin auch die röntgendurchlässige Wand 5 weisen eine rechteckige Form in der Detektorebene auf, wobei das Gehäuse 4 eine erste Höhe in einer Höhenrichtung 6 des Detektormoduls 3 und eine erste Breite in einer Breitenrichtung 7 des Detektormoduls 3 aufweist. Ersichtlich ist die erste Höhe, also die Ausdehnung in der Höhenrichtung 6, größer als die erste Breite, also die Ausdehnung in der Breitenrichtung 7. Der Röntgendetektor 2 weist eine zweite Breite und eine zweite Höhe auf, die ersichtlich jeweils kleiner als die erste Breite und die erste Höhe sind. Über wenigstens einen entsprechenden, hier nur gestrichelt angedeuteten, ansteuerbaren Verschiebeaktor 8 ist der Röntgendetektor 2 innerhalb des Gehäuses 4 sowohl in Höhenrichtung 6 als auch in Breitenrichtung 7 (in der Grundstellung eine Querrichtung) verschiebbar.

Das Detektormodul 2 wird von einer Stativanordnung 9 getragen, die vorliegend einen Sockel 10, der an oder in einem Boden verankerbar ist, sowie eine teleskopartig mittels eines ansteuerbaren Hubaktors 11 höheneinstellbare Säule 12 (im Folgenden Teleskopsäule 12) aufweist. Die zur Verankerung vorgesehene Verankerungsanordnung 13 kann zur statischen Verankerung beispielsweise eine Verschraubung umfassen, optional kann sie jedoch auch, beispielsweise über ein Schienensystem 14, eine Verschiebbarkeit in einer horizontalen Richtung in dem Boden erlauben.

Wie Fig. 2 zu entnehmen ist, ist das Detektormodul 3 über eine Schwenkeinrichtung 15 und eine Kippeinrichtung 16 an der Teleskopsäule 12 befestigt. Die Schwenkeinrichtung 15 erlaubt es, gegebenenfalls manuell und vorliegend in jedem Fall mittels eines ansteuerbaren Schwenkaktors 17, das Detektormodul 3 um eine zu der Detektorfläche senkrechte, in Fig. 2 auch angedeuteten Schwenkachse zu verschwenken, insbesondere ausgehend von der Grundstellung in eine weitere Schwenkstellung, in der das Detektormodul 3 um 90° geschwenkt ist, sodass die lange Kante des Gehäuses 4, also die Höhenrichtung 6 des Detektormoduls 3, nun horizontal verläuft und die kürzere Kante des Gehäuses 4, also die Breitenrichtung 7 des Detektormoduls 3, nun vertikal verläuft.

Die Kippeinrichtung 16 weist entsprechend einen ansteuerbaren Kippaktor 19 auf, um das Detektormodul 3 aus der vertikalen Grundstellung herauskippen zu können, vorliegend um eine horizontale, parallel zur Detektorfläche liegende Kippachse 20. Auch hier kann gegebenenfalls zusätzlich eine manuelle Verkippung ermöglicht sein. Eine Verkippung ist zumindest in eine Horizontalstellung, in der die Detektorfläche und die röntgendurchlässige Wand 5 sich horizontal erstrecken, wobei die röntgendurchlässige Wand 5 dann die Oberseite bildet, möglich. Dann erstrecken sich mithin sowohl die Breitenrichtung 7 des Detektormoduls 3 als auch die Höhenrichtung 6 des Detektormoduls 3 horizontal. Es ist auch eine Verkippung in Zwischenstellungen oder für Spezialaufnahmen in die andere Kipprichtung denkbar.

Optional zeigt Fig. 2 auch eine Linearverstelleinrichtung 21 mit wenigstens einem ansteuerbaren Linearaktor 22 zur Linearverstellung des Detektormoduls 3 in wenigstens einer horizontalen Richtung insbesondere einer zu der Breitenrichtung 7 und der Höhenrichtung 6 in der Grundstellung senkrechten Längsrichtung. Beispielsweise kann ein Teleskoparm verwendet werden.

Fig. 3 zeigt in einer schematischen Querschnittsansicht das Detektormodul 3 genauer. Der Röntgendetektor 2 ist in seinem Innenraum ersichtlich mittels einer Verschiebeeinrichtung verschiebbar angeordnet. Hierbei können grundsätzlich bekannte Linearstellmittel, beispielsweise Spindeltriebe und/oder bevorzugt ein geführter Schlitten, an dem der Röntgendetektor 2 angeordnet ist, verwendet werden. Vorliegend ist in einer Richtung, insbesondere also der Breitenrichtung 7 oder der Höhenrichtung 6, der Schlitten entlang eines Führungsmittels 23 verschiebbar, während dieses Führungsmittel 23 selbst, insbesondere beidseitig, senkrecht zu dessen Verlauf mittels Linearstellmitteln 24 linearverstellbar ist. Denkbar ist es in anderen Ausführungsbeispielen auch, dass ein Schlitten in den oder durch die Linearstellmittel 24 geführt ist, auf dem der Röntgendetektor 2 in einer hierzu senkrechten Richtung mittels der Führungsmittel 23 verschiebbar ist. Der Verschiebeaktor 8 kann beispielsweise mittels Motoren realisiert sein.

Innerhalb des Gehäuses 4, in einer anderen Ebene parallel zu der Detektorebene bewegbar, ist dem Röntgendetektor 2 ein Streustrahlenraster 25 zugeordnet, welches vorliegend in einer dem Röntgendetektor 2 vorgeschalteten Stellung gezeigt ist und mit diesem gemeinsam bewegt werden kann. Vorliegend ist jedoch auch, beispielsweise über eine vergleichbare weitere Verschiebeeinrichtung, eine unabhängige Verschiebbarkeit des Streustrahlenrasters 25 in der Höhenrichtung 6 und der Breitenrichtung 7 möglich, wobei vorliegend nur der entsprechende wenigstens eine ansteuerbare Rasteraktor 26 angedeutet ist. Hierdurch ist es möglich, das Streustrahlenraster 25, wenn es nicht benötigt wird, in eine nicht mit der Detektorfläche überlappende Position zu verfahren. Zur Entnahme, zum Hinzufügen bzw. zum Austausch des Streustrahlenrasters 25 ist vorliegend auch eine Präsentatoranordnung 27 vorgesehen. Die Präsentatoranordnung 27 weist eine aus dem Gehäuse 4 ausfahrbare Schublade 28 (oder sonstige Ausfahrvorrichtung) auf, aus der das Streustrahlenraster 25 entnehmbar bzw. in die es einsetzbar ist. Das Streustrahlenraster 25 kann mittels des wenigstens einen Rasteraktors 26 in eine Präsentatorposition innerhalb der Schublade 28 bzw. Ausfahrvorrichtung bewegt werden, um dort präsentiert zu werden. Die Präsentatoranordnung 27 kann ein Greifmittel (nicht gezeigt) umfassen, um das Streustrahlenraster 25 für das Ausfahren zu greifen.

Es sei angemerkt, dass in dem Gehäuse 4 auch wenigstens eine, hier der Übersichtlichkeit halber nicht näher gezeigte Dosismesskammer angeordnet sein kann.

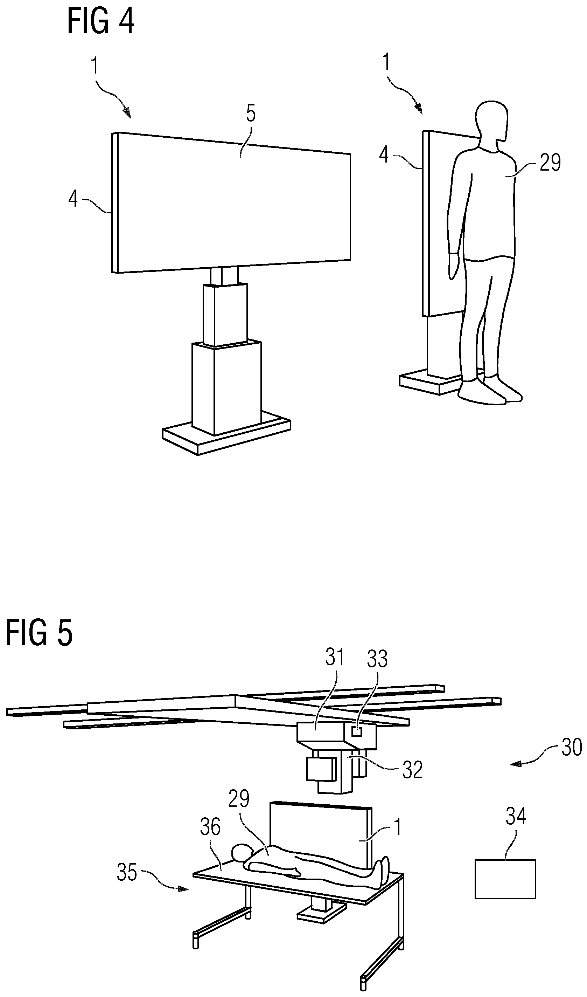

Fig. 4 zeigt zur näheren Erläuterung die Röntgendetektorhalterung 1 in zwei verschiedenen Schwenkstellungen. Das rechte Teilbild zeigt hierbei die Detektorhalterungseinrichtung 1 in der Grundstellung mit einem, wie bei Rasterwandgeräten bekannt, vor der Detektorhalterungseinrichtung 1 stehenden Patienten 29. Zur besseren Unterstützung des Patienten beim Einnehmen und/oder Halten einer Stellung für die Bildaufnahme kann an dem Gehäuse 4 auch wenigstens ein Haltemittel (der Übersichtlichkeit nicht gezeigt) vorgesehen sein.

Das linke Teilbild der Fig. 4 zeigt die Detektorhalterungseinrichtung 1 in der oben bereits diskutierten weiteren Schwenkstellung, also um 90° um die Schwenkachse 18 gedreht. Die weitere Schwenkstellung wurde durch entsprechendes Ansteuern des Schwenkaktors 17 eingenommen. Sie ist insbesondere, wie noch genauer erläutert werden wird, für Lateralaufnahmen bei liegendem Patienten 29 geeignet.

Fig. 5 zeigt ein erstes Ausführungsbeispiel einer erfindungsgemäßen Röntgeneinrichtung 30. Die Röntgeneinrichtung 30 umfasst eine Strahlerhalterungseinrichtung 31 mit einem Röntgenstrahler 32 mit Tiefenblende, die vorliegend an einer Decke des Raumes, in dem die Röntgeneinrichtung 30 installiert ist, montiert ist. Über wenigstens einen ansteuerbaren Strahleraktor 33 kann der Röntgenstrahler 32 mit Tiefenblende in verschiedene Positionen verfahren werden. Neben der Strahlerhalterungseinrichtung 31 umfasst die Röntgeneinrichtung 30 auch die in Bezug auf die Figuren 1 bis 4 beschriebene Detektorhalterungseinrichtung 1 sowie eine Steuereinrichtung 34, wie vorliegend nur schematisch angedeutet ist. Mittels der Steuereinrichtung 34 kann die Aktorik der Strahlerhalterungseinrichtung 31 und der Detektorhalterungseinrichtung 1, insbesondere also der wenigstens eine Verschiebeaktor 8, der Schwenkaktor 17, der Kippaktor 19, der Linearaktor 22, der Hubaktor 11 und der Rasteraktor 26, angesteuert werden. Hinsichtlich des Röntgendetektors 2 sind sechs Freiheitsgrade steuerbar: Die Verschiebbarkeit in der Höhenrichtung 6, die Verschiebbarkeit in der Breitenrichtung 7, die Schwenkbarkeit um die Schwenkachse 18, die Kippbarkeit um die Kippachse 20, die Längsverschiebung mit dem Linearaktor 22 und die Höheneinstellung mittels des Hubaktors 11. Zudem kann die Steuereinrichtung 34 den Röntgenstrahler 32 und den Röntgendetektor 2 zur Aufnahme von Röntgenbildern ansteuern. Durch die Ansteuerung der Aktorik sowie die Steuerung des Aufnahmebetriebs ist eine Automatisierung vieler Workflows möglich.

Die Röntgeneinrichtung 30 umfasst vorliegend auch als mobiles Patientenlagerungsmittel 35 einen einfach ausgestalteten Trolley 36, auf dem ein Patient 29 liegend gelagert werden kann. In der Darstellung der Fig. 5 befindet sich das Detektormodul 3 in der weiteren Schwenkstellung und ist seitlich der mobilen Patientenlagerungseinrichtung 35 angeordnet, sodass Lateralaufnahmen des Patienten 29 angefertigt werden können. Diese können aufgrund der Verwendung der weiteren Schwenkstellung in vielen Positionen entlang der Längsrichtung des Patienten 29 aufgenommen werden, da die Höhenrichtung 6, in der ein größerer Verschiebebereich vorliegt, dieser Längsrichtung entspricht. Beispielsweise kann eine Panoramaaufnahme mit der Aufnahme von mehreren Einzelbildern in der Patientenlängsrichtung durchgeführt werden, wobei die Steuereinrichtung 34 die Bewegung des Röntgendetektors 2 und des Röntgenstrahlers 32 genau wie deren Aufnahmebetrieb steuern kann. Hierbei wird der Röntgendetektor 2 immer korrekt dem Röntgenstrahler 32 nachgeführt, sodass die Röntgenstrahlen des Röntgenstrahlers 32 durch den Röntgendetektor 2 auf dessen Detektorfläche empfangen werden können. Insbesondere sind hierzu die Koordinatensysteme der Halterungseinrichtungen 1, 31 miteinander registriert.

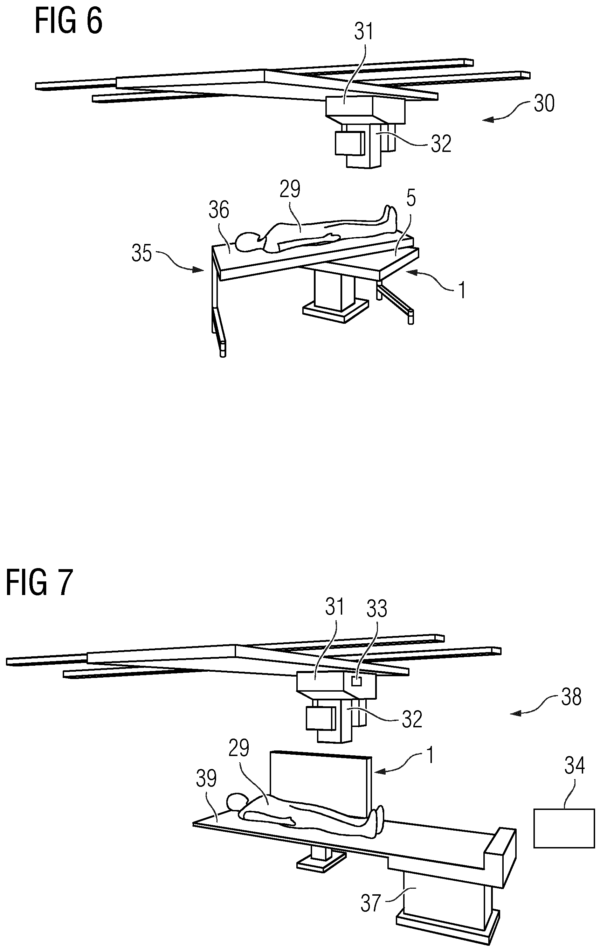

Fig. 6 zeigt die Röntgeneinrichtung 30 in einem weiteren Betriebszustand. In diesem Fall ist das Detektormodul 3 in die Horizontalstellung gekippt. Aufgrund der teleskopierbaren Säule 12 überragt die Stativanordnung 9 das Detektormodul 3 nicht und die Patientenlagerungsfläche der mobilen Patientenlagerungseinrichtung 35 kann problemlos oberhalb der röntgendurchlässigen Wand 5 positioniert werden, um Röntgenaufnahmen bei liegendem Patienten 29 in anterior-posterior-Richtung durchzuführen.

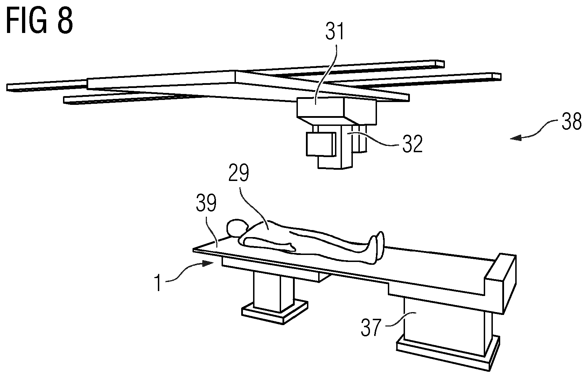

Die Figuren 7 und 8 zeigen ein zweites Ausführungsbeispiel einer Röntgeneinrichtung 38 in entsprechenden Betriebszuständen, wobei im Unterschied zu der Röntgeneinrichtung 30 statt der mobilen Patientenlagerungseinrichtung 35 bei der Röntgeneinrichtung 38 ein fest installierter Patiententisch 37, der jedoch selbst keinerlei Röntgendetektor 2 oder Einschub dafür aufweist, verwendet wird. Ersichtlich lässt sich auch in einem solchen Fall gemäß Fig. 7 die Detektorhalterungseinrichtung 1 für Lateralaufnahmen und gemäß Fig. 8 bei einer Positionierung unterhalb der Tischplatte 39 für anterior-posterior-Aufnahmen bei liegendem Patienten 29 verwenden.

Selbstverständlich kann sowohl bei der Röntgeneinrichtung 30 als auch bei der Röntgeneinrichtung 38 die Detektorhalterungseinrichtung 1 auch für Röntgenaufnahmen bei stehendem Patienten 29, wie in Fig. 4 rechts illustriert, verwendet werden.

Unabhängig vom grammatikalischen Geschlecht eines bestimmten Begriffes sind Personen mit männlicher, weiblicher oder anderer Geschlechteridentität mit umfasst.

## Patentansprüche

1. Detektorhalterungseinrichtung (1) für eine Röntgeneinrichtung (30, 38), aufweisend einen Röntgendetektor (2) und eine Stativanordnung (9), von welcher der Röntgendetektor (2) innerhalb eines Gehäuses (4) eines Detektormoduls (3) getragen wird, wobei der Röntgendetektor (2) eine sich in einer Detektorebene erstreckende Detektorfläche, die hinter einer röntgendurchlässigen Wand (5) des Gehäuses (4) angeordnet ist, aufweist, **dadurch gekennzeichnet, dass** der Röntgendetektor (2) mittels wenigstens eines ansteuerbaren Verschiebeaktors (8) in wenigstens einer Richtung (6, 7) in der Detektorebene innerhalb des Gehäuses (4) bewegbar ist.

2. Detektorhalterungseinrichtung nach Anspruch 1, **dadurch gekennzeichnet, dass** der Röntgendetektor (2) mittels des wenigstens einen Verschiebeaktors (8) in einer Höhenrichtung (6) und in einer Breitenrichtung (7), die senkrecht zueinander stehen, verschiebbar ist.

3. Detektorhalterungseinrichtung nach Anspruch 1 oder 2, **dadurch gekennzeichnet, dass** in dem Gehäuse (4) ein dem Röntgendetektor(2) zugeordnetes Streustrahlenraster (25) vorgesehen ist, welches zumindest mit dem Röntgendetektor (2) bewegbar ist.

4. Detektorhalterungseinrichtung nach Anspruch 3, **dadurch gekennzeichnet, dass** das Streustrahlenraster (25) mittels wenigstens eines Rasteraktors (26) unabhängig von dem Röntgendetektor (2) in der wenigstens einen Richtung (6, 7) bewegbar ist.

5. Detektorhalterungseinrichtung nach Anspruch 4, **dadurch gekennzeichnet, dass** das Detektormodul (3) eine Präsentatoranordnung (27) zum Einsetzen und Entnehmen des Streustrahlenrasters (25) aufweist.

6. Detektorhalterungseinrichtung nach einem der vorangehenden Ansprüche, **dadurch gekennzeichnet, dass** zumindest in einer Grundstellung die Detektorfläche vertikal ausgerichtet ist.

7. Detektorhalterungseinrichtung nach einem der vorangehenden Ansprüche, **dadurch gekennzeichnet, dass** die Stativanordnung (9) eine teleskopartig mittels eines ansteuerbaren Hubaktors (11) höheneinstellbare Säule (12) umfasst, die das Detektormodul (3) trägt.

8. Detektorhalterungseinrichtung nach Anspruch 7, **dadurch gekennzeichnet, dass** das Detektormodul (3) über eine Linearverstelleinrichtung (21) mit wenigstens einem ansteuerbaren Linearaktor (22) in wenigstens einer horizontalen Richtung verstellbar mit der Säule (12) gekoppelt ist.

9. Detektorhalterungseinrichtung nach einem der vorangehenden Ansprüche, **dadurch gekennzeichnet, dass** sie eine Verankerungsanordnung (13), insbesondere umfassend ein Schienensystem (14), zur in zumindest einer horizontalen Richtung verschiebbaren Verankerung der Stativanordnung (9) in einem Boden aufweist.

10. Detektorhalterungseinrichtung nach einem der vorangehenden Ansprüche, **dadurch gekennzeichnet, dass** das Detektormodul (3) mittels einer Kippeinrichtung (16) zumindest zwischen einer oder der Grundstellung, in der die Detektorfläche vertikal ausgerichtet ist, und einer Horizontalstellung, in der die Detektorfläche horizontal ausgerichtet ist, kippbar mit der Stativanordnung (9) gekoppelt ist, wobei die Kippeinrichtung (16) insbesondere einen ansteuerbaren, die Kippung bewirkenden Kippaktor (19) aufweist.

11. Detektorhalterungseinrichtung nach einem der vorangehenden Ansprüche, **dadurch gekennzeichnet, dass** das Detektormodul (3) über eine Schwenkeinrichtung (15), die ein Verschwenken des Detektormoduls (3) um eine zur Detektorfläche senkrechte Schwenkachse (18) erlaubt, mit der Stativanordnung (9) gekoppelt ist, wobei die Schwenkeinrichtung (15) insbesondere einen ansteuerbaren, die Verschwenkung bewirkenden Schwenkaktor (17) aufweist.

12. Detektorhalterungseinrichtung nach einem der vorangehenden Ansprüche, **dadurch gekennzeichnet, dass** das Detektormodul (3), insbesondere das Gehäuse (4), wenigstens einen Haltegriff für einen Patienten (29) aufweist.

13. Detektorhalterungseinrichtung nach einem der vorangehenden Ansprüche, **dadurch gekennzeichnet, dass** das Gehäuse (4) in der Detektorebene eine Ausdehnung von 50 bis 100 mal 50 bis 150 cm aufweist und/oder der Röntgendetektor (2) in der Detektorebene eine Ausdehnung von 20 bis 50 mal 20 bis 80 cm aufweist.

14. Röntgeneinrichtung (30, 38), aufweisend eine Strahlerhalterungseinrichtung (31) mit wenigstens einem Röntgenstrahler (32) und wenigstens einem ansteuerbaren Strahleraktor (33) zur Positionierung des Röntgenstrahlers (32), eine Detektorhalterungseinrichtung (1) nach einem der vorangehenden Ansprüche und eine Steuereinrichtung (34).

15. Röntgeneinrichtung nach Anspruch 14, **dadurch gekennzeichnet, dass** sie ferner wenigstens einen Patiententisch (37) und/oder wenigstens eine mobile Patientenlagerungseinrichtung (35) aufweist.
